# EUROPEAN PATENT APPLICATION

(11) **EP 4 063 860 A1**
(43) Date of publication of application: **28.09.2022**
(21) Application number: 20890068.8
(22) Date of filing: 20.11.2020
(51) Int. Cl.: G01N 33/53, G01N 33/543, G01N 33/545

(54) **REAGENT FOR MEASURING 25-HYDROXY VITAMIN D AND METHOD FOR MEASURING 25-HYDROXY VITAMIN D**

(30) Priority: 22.11.2019 JP 2019211337
(71) Applicant: Sekisui Medical Co., Ltd., Tokyo 103-0027 (JP)
(72) Inventor: YAMAMOTO Mitsuaki, Tokyo 103-0027 (JP); OTA Mieko, Tokyo 103-0027 (JP); NISHIO Tomohisa, Tokyo 103-0027 (JP)
(74) Representative: Hoffmann Eitle
(86) International application number: PCT/JP2020/043324
(87) International publication number: WO 2021/100841

(57) **Abstract**

The present invention addresses the problem of providing a method for measuring 25-hydroxy vitamin D and a measurement reagent, the method and the reagent being based on a competitive immunoassay. The present invention provides a reagent for measuring 25-hydroxy vitamin D based on a competitive immunoassay, which includes at least the following composition: (1) a Vitamin D derivative represented by the chemical formula (I) and/or (II), (2) an anti-25 hydroxy vitamin D antibody. In addition, the present invention provides a reagent for measuring vitamin D based on a competitive latex turbidimetric immunoassay (competitive LTIA) in which, in particular, a vitamin D derivative or an anti-25-hydroxy vitamin D antibody is immobilized on latex.

## Description

### Technical Field

The present invention relates to a reagent for measuring 25-hydroxy vitamin D and a method for measuring 25-hydroxy vitamin D. In particular, the present invention relates to a reagent for measuring 25-hydroxy vitamin D and a method for measuring 25-hydroxy vitamin D, the reagent and the method based on a competitive immunoassay.

### Background Art

Vitamin D is an important substance with many implications in the biological processes of the human and animal body. Physiologically active vitamin D is known to regulate calcium absorption from the intestinal tract and bone calcification in particular. It also has the effect of increasing the serum calcium concentration. A deficiency or excess in vitamin D is known to have various consequences, and in particular, vitamin D deficiency is known to lead to serious diseases such as osteoporosis and rickets. Therefore, the quantification of vitamin D in the body is important for revealing a potential deficiency or excess.

Vitamin D is present in the living body in two forms, namely vitamin D2 (ergocalciferol) and vitamin D3 (cholecalciferol) as represented in Figure 1.

Vitamin D2 is an exogenous vitamin D obtained from food, and vitamin D3 is an endogenous vitamin D produced by the action of the ultraviolet rays from the sun on the skin. These vitamin Ds bind to the vitamin D-binding protein and are transported to the liver by this protein, where the carbon at position 25 is hydroxylated in the liver to form 25-hydroxy vitamin Ds (25(OH)VDs). Furthermore, some of them are hydroxylated at the carbon at position 1 in the kidneys to produce the active forms, 1,25(OH)₂VDs.

The above two-step metabolism of vitamin D occurring in the living body will be described with reference to Figure 1. In the first step, vitamin D2 or vitamin D3 is metabolized into 25-hydroxy vitamin D2 or 25-hydroxy vitamin D3 (hereinafter, sometimes referred to as 25(OH)VD2 and 25(OH)VD3, respectively. These are collectively referred to as 25-hydroxy vitamin D (hereinafter sometimes referred to as 25(OH)VD)). Then, 25(OH)VD2 or 25(OH)VD3 is metabolized into 1,25-dihydroxy vitamin D2 or 1,25-dihydroxy vitamin D3 (hereinafter, sometimes simply referred to as 1,25(OH)₂D2 and 1,25(OH)₂D3, respectively, and collectively referred to as 1,25(OH)₂VD).

Among these metabolites, the stable 25(OH)VD is a useful index since it reflects well the vitamin D sufficiency in the blood.

Here, the following methods are known as methods for measuring 25(OH)VD.

"Lumipulse (registered trademark) 25-hydroxyvitamin D" is known as a reagent for a method for measuring 25(OH)VD in serum or plasma by chemiluminescent enzyme immunoassay (CLEIA). The present method is a method of measuring the 25(OH)VD concentration in a sample by measuring the amount of luminescence by an enzymatic reaction through a first reaction of forming an immune complex with anti-25(OH)VD monoclonal antibody-bound ferrite particles and 25(OH)VD in the sample, and a second reaction of forming an immune complex with the above complex and enzyme-labeled anti-25(OH)VD immune complex monoclonal antibodies. The present method requires a washing step after the first reaction and after the second reaction, and is a complex process.

In addition, Patent Literature 1 discloses an immunoassay of 25(OH)VD using a disposable cone. In the present method, a pretreated sample is mixed with enzyme-labeled anti-vitamin D antibodies in a well, incubated, then transferred to a vitamin D-immobilized cone, and further incubated. During this time, the antigen in the sample, 25(OH)VD, and the vitamin D immobilized on the cone compete for the antibody site of the enzyme-labeled anti-vitamin D antibody, and a complex of the vitamin D immobilized on the cone and the enzyme-labeled anti-vitamin D antibody can be detected by removing the nonimmobilized products by washing. Since the concentration of the complex is inversely proportional to the antigen concentration in the sample, 25(OH)VD in the sample can be calculated. However, the present method also requires a washing step, and is a complex process as described above.

### Citation List

### Patent Literature

Patent Literature 1: Japanese Translation of PCT International Application Publication No. 2016-531306

### Summary of Invention

### Technical Problem

As described above, the conventional method for measuring 25(OH)VD is a complex process, and there was no method based on latex turbidimetric immunoassay, which is a homogeneous measurement method which does not require a washing step.

The latex turbidimetric immunoassay (hereinafter, sometimes referred to as LTIA) is a method for measuring a test substance using latex particles on which antigens or antibodies are immobilized, and is widely used in the field of clinical testing. LTIA can be roughly classified into a method of reacting latex particles on which antibodies against a test substance are immobilized, with an antigen, which is the test substance, to form a sandwich-type immune complex, and measuring the test substance (antigen) from the degree of agglutination of the latex particles associated with the formation of the immune complex (hereinafter, sometimes referred to as sandwich LTIA), and a method of inhibiting the formation of an immune complex between the latex particles and the antigen (test substance) by the competition between a synthetic antigen and the antigen in the test sample (test substance) in the presence of latex particles on which an antibodies are immobilized, and measuring the test substance (antigen) from the degree of inhibition of agglutination (agglutination inhibition) of the latex particles associated with the inhibition of formation of the immune complex (hereinafter, sometimes referred to as competitive LTIA, or the like).

The present invention addresses the problem of providing a method for measuring 25(OH)VD by competitive LTIA and a measurement reagent. Furthermore, the present invention also addresses the problem of providing a method for measuring 25(OH)VD and a measurement reagent, the method and the reagent being based not only on competitive LTIA, but also on competitive immunoassay. Solution to Problem

The inventors have prepared a polyvalent antigen in which two or more 25(OH)VDs are bound to a carrier such as a protein in competitive LTIA, and investigated a measurement by competitive LTIA in which the maximum agglutination occurs when the 25(OH)VD concentration in blood is zero, and the amount of agglutination decreases as the concentration of 25(OH)VD in the blood increases. That is, the inventors investigated a measurement system using a two-reagent kit containing the polyvalent antigen in a first reagent and an anti-25(OH)VD antibodysensitized latex in a second reagent. Here, when the 25(OH)VD, which is the target of measurement, was derivatized to be the polyvalent antigen, the detection sensitivity was not sufficiently satisfactory. However, the inventors discovered that the measurement sensitivity surprisingly increases when a structure obtained by derivatizing vitamin D represented by the chemical formula (I), which is different from the 25(OH)VD to be measured, is used as a competing polyvalent antigen.

In addition, the inventors found that this found action is not limited to competitive LTIA and can also be applied to competitive immunoassay, and completed the present invention.

More specifically, the present invention has the following constitution:
<1> A reagent for measuring 25-hydroxy vitamin D based on a competitive immunoassay, which includes at least the following composition:
   (1) A vitamin D derivative represented by the following chemical formula (I) and/or (II); and
   (2) An anti-25 hydroxy vitamin D antibody, 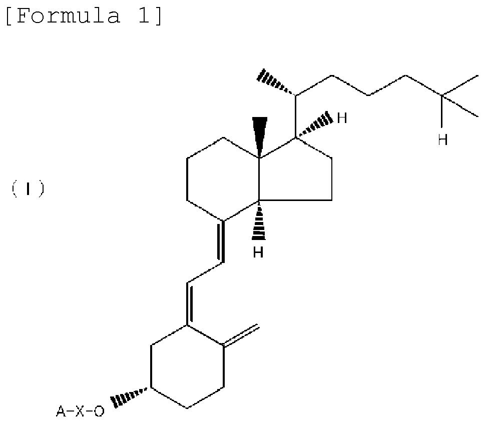 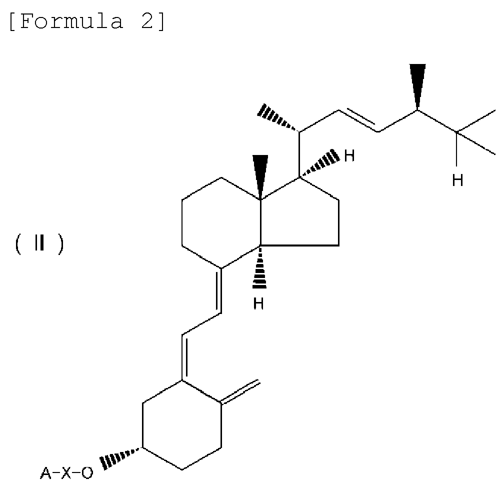 wherein A represents a tracer group capable of chemically binding to a carrier with high affinity, and X indicates a hydrocarbon group having a chain length of 3 to 20, which is unsubstituted or substituted with a hetero atom.
<2> The reagent for measuring 25-hydroxy vitamin D according to <1>, wherein the A is selected from an amino group, a carboxyl group, a sulfhydryl group, biotin, digoxygenin, tyrosine, FITC-substituted tyrosine, substituted amino acids, amino acid and peptide sequences, FITC, proteins and peptides, A-proteins, G-proteins, and vitamin D derivatives.
<3> The reagent for measuring 25-hydroxy vitamin D according to <1> or <2>, wherein the vitamin D derivative represented by the chemical formula (I) or (II) is in a configuration in which two or more molecules bind to a carrier via A and form a polyvalent antigen.
<4> The reagent for measuring 25-hydroxy vitamin D according to any one of <1> to <3>, wherein the measurement principle of the competitive immunoassay is a competitive immunoassay selected from RIA, EIA, LTIA, and CLEIA.
<5> The reagent for measuring 25-hydroxy vitamin D according to <4>, wherein the competitive immunoassay is competitive latex turbidimetric immunoassay (competitive LTIA).
<6> The reagent for measuring 25-hydroxy vitamin D according to any one of <1> to <5>, wherein 25-hydroxy vitamin D is the sum of 25-hydroxy vitamin D2 and 25-hydroxy vitamin D3, the vitamin D derivative is a vitamin D2 derivative and/or vitamin D3 derivative, and the anti-25-hydroxy vitamin D antibody is an anti-25-hydroxy vitamin D2 and/or anti-25-hydroxy vitamin D3 antibody.
<7> The reagent for measuring 25-hydroxy vitamin D according to any one of <1> to <6>, wherein the vitamin D3 derivative is a compound represented by the following chemical formula (III): 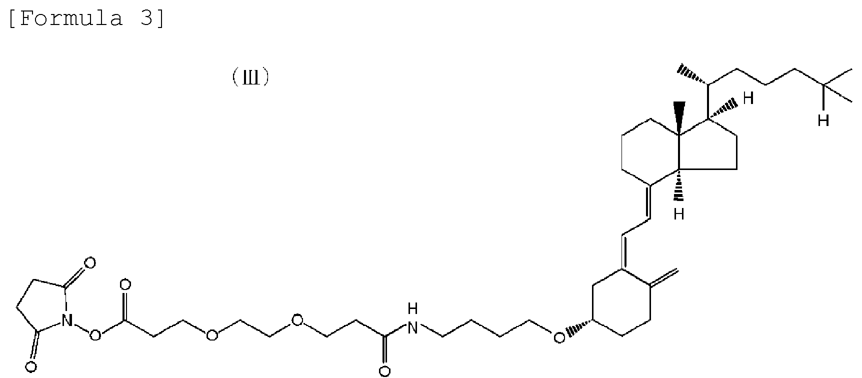
<8> The reagent for measuring 25-hydroxy vitamin D according to any one of <1> to <7>, wherein either (1) or (2) is immobilized on an insoluble carrier.
<9> The reagent for measuring 25-hydroxy vitamin D according to any one of <1> to <8>, which is a reagent for an automated analyzer.
<10> A method for measuring 25-hydroxy vitamin D based on a competitive immunoassay, which includes at least the following steps:
   (1) A step of bringing a sample into contact with anti-25-hydroxy vitamin D antibodies in the presence of a vitamin D derivative represented by the chemical formula (I) and/or (II): 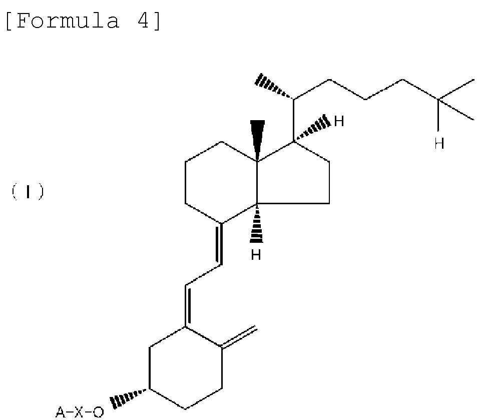 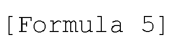 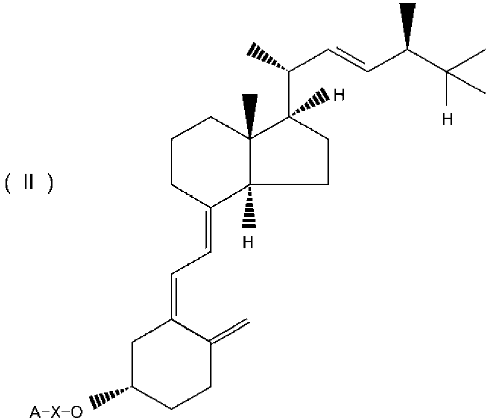 wherein A represents a tracer group capable of chemically binding to a carrier with high affinity; and X indicates a hydrocarbon group having a chain length of 3 to 20, which is unsubstituted or substituted with a hetero atom; and
   (2) A step of measuring the degree of inhibition of the antigen-antibody reaction between the vitamin D derivative and the anti-25-hydroxy vitamin D antibodies according to the 25-hydroxy vitamin D in the sample.
<11> The method for measuring 25-hydroxy vitamin D according to <10>, wherein A is selected from an amino group, a carboxyl group, a sulfhydryl group, biotin, digoxygenin, tyrosine, FITC-substituted tyrosine, substituted amino acids, amino acid and peptide sequences, FITC, proteins and peptides, A-proteins, G-proteins, and vitamin D derivatives.
<12> The method for measuring 25-hydroxy vitamin D according to <10> or <11>, wherein the vitamin D derivative represented by the chemical formula (I) or (II) is in a configuration in which two or more molecules bind to a carrier via A and form a polyvalent antigen.
<13> The method for measuring 25-hydroxy vitamin D according to any one of <10> to <12>, wherein the measurement principle of the competitive immunoassay is a competitive immunoassay selected from RIA, EIA, LTIA, and CLEIA.
<14> The method for measuring 25-hydroxy vitamin D according to <13>, wherein the competitive immunoassay is competitive latex turbidimetric immunoassay (competitive LTIA).
<15> The method for measuring 25-hydroxy vitamin D according to any one of <10> to <14>, wherein 25-hydroxy vitamin D is the sum of 25-hydroxy vitamin D2 and 25-hydroxy vitamin D3, the vitamin D derivative is a vitamin D2 derivative and/or vitamin D3 derivative, and the anti-25-hydroxy vitamin D antibody is anti-25-hydroxy vitamin D2 and/or anti-25-hydroxy vitamin D3 antibody.
<16> The method for measuring 25-hydroxy vitamin D according to any one of <10> to <15>, wherein the vitamin D3 derivative is a compound represented by the following chemical formula (III): 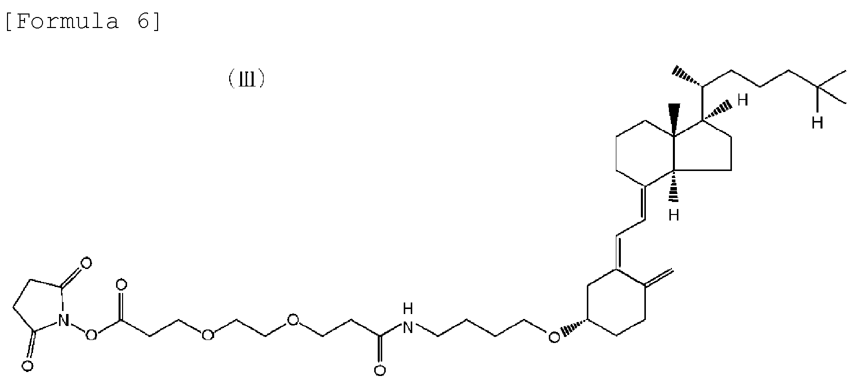
<17> The method for measuring 25-hydroxy vitamin D according to any one of <10> to <16>, wherein either (1) or (2) is immobilized on an insoluble carrier.
<18> The method for measuring 25-hydroxy vitamin D according to any one of <10> to <17>, which uses an automated analyzer.
<19> A polyvalent antigen used in the method for measuring 25-hydroxy vitamin D3 based on a competitive immunoassay, wherein a vitamin D3 derivative represented by the following chemical formula (III) is immobilized on a carrier:

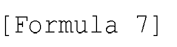

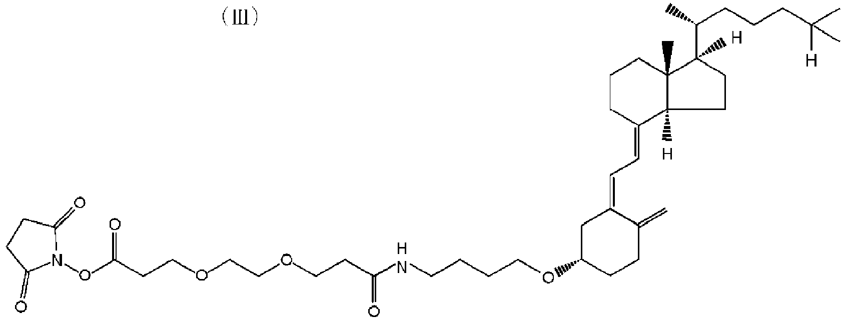

### Advantageous Effects of Invention

According to the present invention, it has become possible to measure 25(OH)VD in a sample even at a low concentration by competitive LTIA. In addition, it is possible to accurately assess the state of 25(OH)VD concentration in a patient sample by increasing the detection sensitivity, which can contribute to assessing the pathological condition. In addition, competitive LTIA can be performed by an automated analyzer which is a general-purpose equipment, and therefore allows to measure a large number of samples in a short time.

Furthermore, in the method for measuring hydroxy vitamin D by immunoassay such as the conventional chemiluminescent enzyme immunoassay (CLEIA), it is also possible to measure the hydroxy vitamin D in the sample up to a low concentration range by using the polyvalent antigen of the present invention.

In addition, an effect of improving the reproducibility of the measurement can be expected even in the low concentration range that was already measurable.

### Brief Description of Drawings

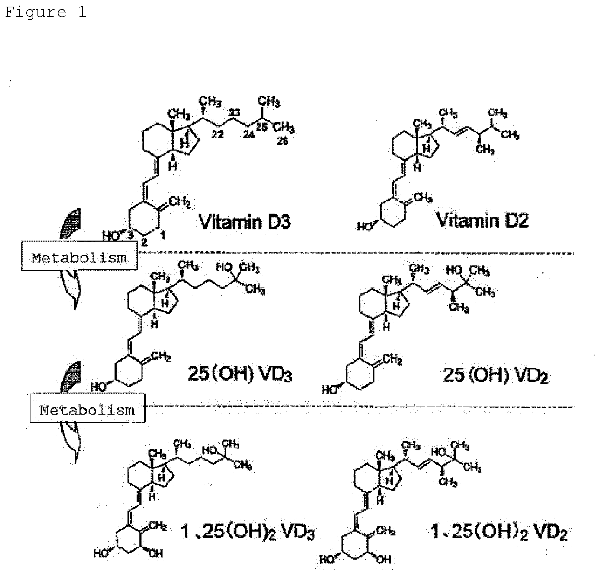

[Figure 1] Figure 1 is a conceptual schematic diagram showing the metabolism of vitamin D in the living body.
[Figure 2] Figure 2 is a graph representing the measurement sensitivity (mAbs.) in each dilution series when a 25(OH)VD3 derivative-BSA complex (Comparative Example 2) or a VD3 derivative-BSA complex (Example 2) was added to the measurement reagent in competitive LTIA, as a relative sensitivity (%) with respect to a zero 25(OH)VD3 concentration.

### Description of Embodiments

### (Measurement Method/Measurement Reagent)

The method for measuring 25(OH)VD of the present invention is a measurement method based on a competitive immunoassay and includes at least the following steps (1) and (2):
(1) A step of bringing a sample into contact with anti-25-hydroxy vitamin D antibodies (hereinafter, sometimes referred to as anti-25(OH)VD) in the presence of a vitamin D derivative represented by the following chemical formula (I) and/or (II)
(2) A step of measuring the degree of inhibition of the antigen-antibody reaction between the vitamin D derivative and the anti-25(OH)VD antibodies according to the 25(OH)VD concentration in the sample

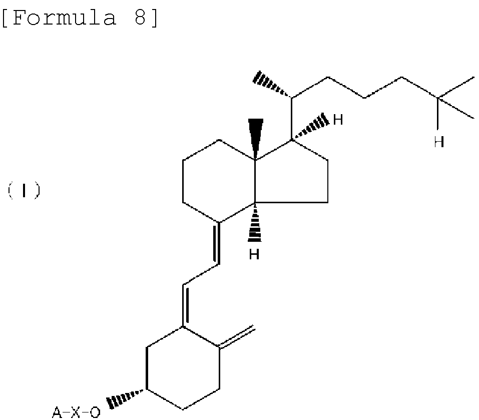

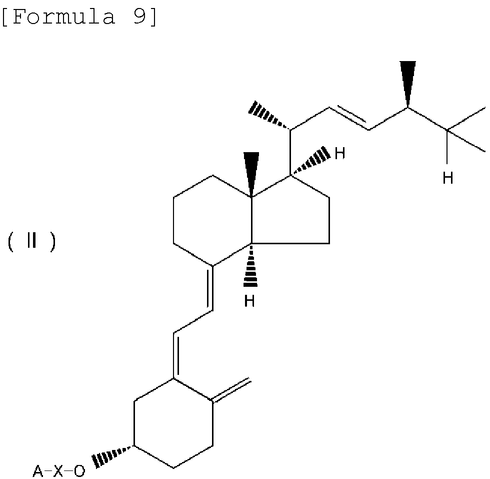

Here, A represents a tracer group capable of chemically binding to a carrier with high affinity.

X indicates a hydrocarbon group having a chain length of 3 to 20, which is unsubstituted or substituted with a hetero atom.

Examples of the measurement principle of the competitive immunoassay include RIA, EIA, LTIA, and CLEIA. It is desirable that either the vitamin D derivative or the anti-25(OH)VD antibody be immobilized on an insoluble carrier. Note that a configuration forming a polyvalent antigen immobilized on a carrier such as a protein is typical as an example of the vitamin D derivative of the present invention. Hereinafter, a case where a vitamin D derivative forming a polyvalent antigen is used will be described.

### (1) Application to Competitive LTIA

A method for measuring 25(OH)VD by a competitive latex immunoassay (competitive LTIA) using latex particles as an insoluble carrier will be described. For example, when the sample and the latex particles on which the anti-25(OH)VD antibodies are immobilized are brought into contact with each other in the presence of a polyvalent antigen in which a vitamin D derivative is immobilized on a carrier ((1-1) below), the degree of agglutination of the latex particles decreases according to the concentration of 25(OH)VD in the sample, and therefore it is possible to measure 25-hydroxy vitamin D by observing the degree of latex agglutination optically or electrochemically.

An example of reagent composition for competitive LTIA is shown below.

### <Example of Reagent Composition>

### (1-1) Antibody Sensitization System

Latex particles on which anti-25(OH)VD antibodies are immobilized
Polyvalent antigen in which a vitamin D derivative is immobilized on a carrier

### (1-2) Antigen Sensitization System

Free anti-25(OH)VD antibodies (secondary antibodies may be added)
Latex particles on which vitamin D derivatives are immobilized

Note that secondary antibodies may be added to the test solution containing the free anti-25(OH)VD antibodies.

### (2) Application to ELISA

A method for measuring 25-hydroxy vitamin D by ELISA using a plate as the insoluble carrier will be described. For example, when a polyvalent antigen, in which a vitamin D derivative is immobilized on a carrier, is immobilized on a well of a plate and a sample and free anti-25(OH)VD antibodies are added to the well of the plate, a competition for the anti-25(OH)VD antibody occurs between the 25(OH)VD in the sample and the immobilized vitamin D derivative, and therefore it is possible to measure 25(OH)VD by observing optically or electrochemically the degree of inhibition of immune reaction according to the concentration of 25(OH)VD in the sample.

An example of reagent composition for ELISA is shown below.

### <Example of Reagent Composition>

A plate on which vitamin D derivatives are immobilized

### Free anti-25(OH)VD antibodies

Note that secondary antibodies may be added to the test solution containing the free anti-25(OH)VD antibodies.

### (3) Application to chemiluminescence using magnetic particles

A method for measuring 25(OH)VD by chemiluminescence using magnetic particles as an insoluble carrier will be described. For example, when the sample and the magnetic particles on which the anti-25(OH)VD antibodies are immobilized are brought into contact with each other in the presence of a labeled polyvalent antigen in which a vitamin D derivative is immobilized on a carrier ((3-1) below), the complex of the antibodies of the magnetic particles and the labeled polyvalent antigen decreases according to the concentration of 25(OH)VD in the sample, and therefore it is possible to measure 25(OH)VD by optically observing the labeling of the recovered magnetic particles. When the sample and the labeled anti-25(OH)VD antibodies are brought into contact with each other in the presence of magnetic particles on which a vitamin D derivative is immobilized (or also includes magnetic particles on which polyvalent antigens are immobilized in which a vitamin D derivative is immobilized on a carrier)((3-2) below), the complex of the magnetic particles and the labeled antibodies decreases according to the concentration of 25(OH)VD in the sample, and therefore it is possible to measure 25(OH)VD by optically observing the labeling of the recovered magnetic particles.

An example of reagent composition for immunoassay using magnetic particles is shown below.

### <Example of Reagent Composition>

### (3-1) Antibody Sensitization System

Magnetic particles on which anti-25(OH)VD antibodies are immobilized
Labeled polyvalent antigen in which a vitamin D derivative is immobilized on a carrier

### (3-2) Antigen Sensitization System

Labeled anti-25(OH)VD antibodies
Magnetic particles on which a vitamin D derivative is immobilized (or including magnetic particles on which polyvalent antigens, in which a vitamin D derivative is immobilized on a carrier, are immobilized)

### (Sample)

The origin of the sample is not particularly limited, and it may be a biological sample derived from an organism, as well as an environmental sample or the like. Examples of the organism from which the biological sample is derived include an animal such as a mammal (eg, human, monkey, mouse, rat, rabbit, cow, pig, horse, goat, and sheep), and a bird (eg, chicken), an insect, a microorganism, a plant, a fungus, and a fish, but it is preferably a mammal, a fungus, or a fish, more preferably a mammal, and still more preferably a human.

The biological sample may be blood itself or a blood-related sample such as whole blood, serum, and plasma, which are samples derived from blood, saliva, urine, breast milk, tissue or cell extract, or a mixture thereof, but among these, a blood-related sample is preferable.

Examples of the environmental sample include a sample derived from soil, seawater, or freshwater.

The sample is sometimes used after appropriately diluting, filtering, or the like.

### (Target of Measurement)

In the present description, the target of measurement is 25-hydroxy vitamin D (25(OH)VD) which is hydroxylated at position 25. That is, any of 25(OH)VD2, 25(OH)VD3, or the sum of thereof is a target of measurement. In the present description, when 25-hydroxy vitamin D (25(OH)VD) is indicated, it means that any of 25(OH)VD2, 25(OH)VD3, or the sum thereof is included unless otherwise specified. When measuring the sum, the vitamin D derivative of the present invention is a vitamin D2 derivative and/or vitamin D3 derivative, and the anti-25-hydroxy vitamin D antibody is an anti-25-hydroxy vitamin D2 and/or anti-25-hydroxy vitamin D3 antibody. Each vitamin D derivative and anti-25-hydroxy vitamin D antibody will be described below.

### (Vitamin D Derivative)

The vitamin D derivative in the present invention refers to a vitamin D that has been chemically modified to bind to the carrier surface, and corresponds to a vitamin D to which a hydrocarbon group and a tracer are bound.

Examples of the vitamin D3 derivative and the vitamin D2 derivative include the compounds represented by the following general formulas (I) and (II):

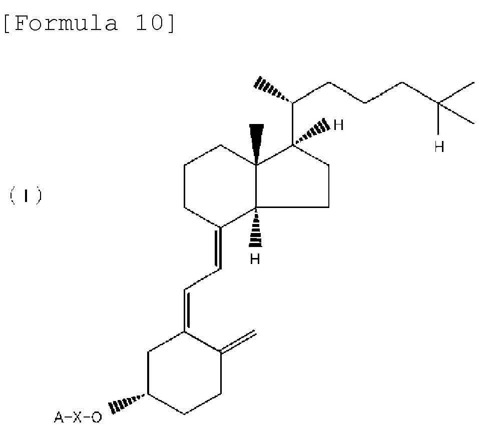

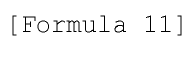

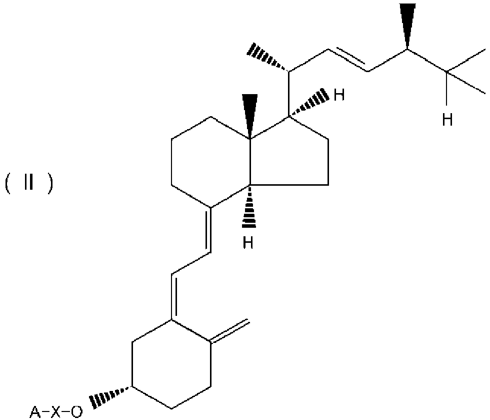

Here, A represents a tracer group capable of chemically binding to a carrier with high affinity.

Specific examples of the A include an amino group, a carboxyl group, a sulfhydryl (-SH) group, biotin, digoxygenin, tyrosine, FITC-substituted tyrosine, a substituted amino acid, an amino acid and peptide sequence, FITC, a peptide, an A-protein, a G-protein, and a vitamin D derivative. In addition, these tracer groups may be activated.

X indicates a hydrocarbon group having a chain length of 3 to 20, which is unsubstituted or substituted with a hetero atom.

Note that examples of heteroatoms include atoms such as S, O, N, and P.

Examples of the carrier include a protein and an insoluble carrier. Examples of the insoluble carrier include a metallic particle, a latex particle, and a plate, and examples of the protein include BSA and vitamin D-binding protein.

When the carrier is a protein, A can be bound to the carrier by using an amino group, a carboxyl group, or a sulfhydryl group as the terminal structure of A.

In addition, the amino group, carboxyl group, or sulfhydryl group can be chemically bound to any carrier protein by further activating it using a known "protein cross-linking agent" or "labeling agent".

Examples of the combination of the structures of the binding site of the carrier protein and the binding site of A include the following.

**[Table 1]**

| Structure of Binding Site of Carrier Protein | Structure of Binding Site of A |
|---|---|
| Carboxyl Group | Amino Group |
| Amino Group | Carboxyl Group |
| Amino Group | Sulfhydryl Group |
| Sulfhydryl Group | Sulfhydryl Group |
| Sulfhydryl Group | Amino Group |

Examples of the above protein cross-linking agent and labeling agent include a biotin labeling agent, a bivalent reagent, a protein labeling reagent, and a derivatization reagent for HPLC, and typical examples of the activation method include the following methods.
(i) Method of converting carboxylic acid into carbodiimide It is a method of activation using a reagent such as DCC (Dicyclohexylcarbodiimide) and EDC (1-Etyl-3-(3-dimethylaminopropyl)carbodiimide) .
(ii) Method of converting carboxylic acid into an N-hydroxysuccinimide-activated ester (Examples described below)
   A method in which NHS (N-hydroxysuccinimide) is allowed to act after EDC.
(iii) Method of converting carboxylic acid into imide type
   A method using DMP (Dimethyl pimelimidate).
(iv) Method of coupling a sulfhydryl group with a maleimide group

In addition, even when the carrier is an insoluble carrier, metallic particles, or the like, it is possible to obtain a conjugate (complex) of the carrier and the vitamin derivative by activating the surface of the carrier or particles to form a structure capable of binding to A.

In the vitamin D3 derivative represented by the chemical formula (II), the case where A is an activated carboxyl group is shown as the following chemical formula (III) :

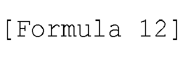

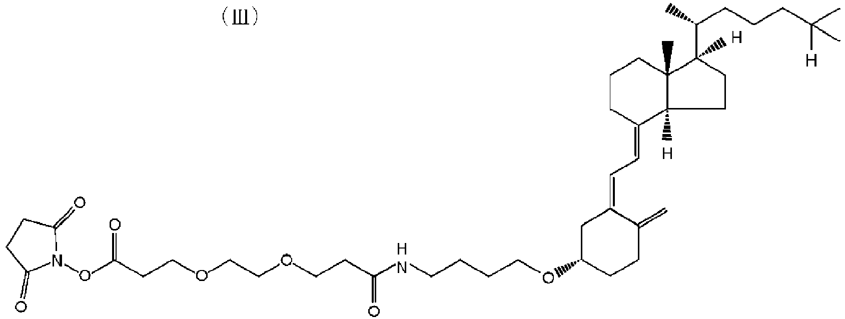

The measurement sensitivity of the competitive immunoassay is considered to depend on the balance between the affinity of the antibody for the measurement target (free 25(OH)VD in the sample) and the affinity for the polyvalent antigen, whereas, in the present invention, it is considered that intentionally reducing the affinity of the anti-25(OH)VD antibody for the polyvalent antigen proportionally increases the affinity for the free antigen 25(OH)VD, which allows detection even at a low concentration of free antigen 25(OH)VD. Since it is usually considered that those skilled in the art will use as the competing polyvalent antigen a polyvalent antigen having the same structure as the free antigen 25(OH)VD at least in the epitope portion, it was surprising that the detection sensitivity increased as a result of trying to use a non-hydroxy form instead of a hydroxy derivative. Furthermore, since the action of enabling the detection of the free antigen 25(OH)VD at a low concentration range by intentionally reducing the affinity of such anti-25(OH)VD antibody for the polyvalent antigen, and proportionally increasing the affinity for the free antigen 25(OH)VD, is an action applicable not only to a competitive immunoassay but also to the competitive immunoassay in general, its application to a competitive immunoassay is also included in the scope of the present invention.

### (Polyvalent Antigen)

The polyvalent antigen used in the present invention refers to two or more vitamin D derivatives of the present invention that are bound to the surface of a carrier.

The vitamin D derivative as the polyvalent antigen of the present invention includes a VD3 derivative represented by the formula (I), a VD2 derivative represented by the formula (II), or a combination thereof, depending on the affinity of the anti-25-hydroxy vitamin D antibody used for the measurement.

The carrier used for the polyvalent antigen is not particularly limited, but a polymer is preferable, and specific examples thereof include a protein such as bovine serum albumin (BSA), keyhole-limpet hemocyanin (KLH), Blue Carrier Protein (BCP), and ovalbumin (OVA). In particular, BSA is preferable in the present invention.

### (Pretreatment)

25(OH)VD is known to be strongly bound to the binding protein (DBP) in the blood. Therefore, in order to accurately measure 25(OH)VD using the antigen-antibody reaction, a dissociation operation (pretreatment) between vitamin D and DBP is required. As such a pretreatment, it is desirable to pretreat by a well-known method such as an acid, a protein denaturant, a surfactant, a denaturant such as a hydrolytic enzyme, or an organic solvent. In addition to these pretreatments, an operation such as centrifugation, extraction, filtration, precipitation, heating, freezing, refrigeration, and agitation is sometimes performed as necessary.

### (Antibody Against 25-Hydroxy Vitamin D)

In the present invention, the anti-25(OH)VD antibody includes a polyclonal antibody or a monoclonal antibody against the 25(OH)VD or 25(OH)VD derivative to be measured, as well as a functional fragment thereof. That is, if the target of measurement is 25(OH)VD2, it includes a polyclonal antibody or a monoclonal antibody against 25(OH)VD2 or 25(OH)VD2 derivatives, as well as a functional fragment thereof. In addition, if the target of measurement is 25(OH)VD3, it includes a polyclonal antibody or a monoclonal antibody against the 25(OH)VD3 or 25(OH)VD3 derivative, as well as a functional fragment thereof. In addition, if the target of measurement is the sum of 25(OH)VD2 and 25(OH)VD3, it includes a polyclonal antibody or a monoclonal antibody that acts on both 25(OH)VD2 or 25(OH)VD2 derivatives, and 25(OH)VD3 or 25(OH)VD3 derivatives, as well as a functional fragment thereof.

A polyclonal antibody can be obtained by recovering the purified target antibody by immunizing an animal with the 25(OH)VD or 25(OH)VD derivative to be tested bound to a carrier protein as the immunogen and obtaining the serum of this animal, then separating the target antibody from the other components of the serum. As the carrier protein, BSA, KLH (keyhole-limpet hemocyanin) and the like can be used.

The monoclonal antibody can be obtained by a hybridoma technique well known to those skilled in the art, or may be a recombinant antibody obtained by genetic engineering using a technique well known to those skilled in the art.

Examples of the functional fragment of the antibody include F(ab')₂ and Fab', which are fragments having an antigen-antibody reaction activity. The functional fragments of these antibodies can be produced by treating the full-length antibodies obtained as described above with a proteolytic enzyme (for example, pepsin and papain).

### (Insoluble Carrier)

The insoluble carrier used in the present invention is an insoluble carrier capable of carrying an anti-25(OH)VD antibody or a polyvalent antigen, and which by bringing it into contact with a sample, allows the measurement of 25(OH)VD in the sample. Examples thereof include a solid phase such as a particle and a plate. Examples of the particle include a latex particle, a magnetic particle, and a metallic particle.

### (Latex Particle)

Examples of the latex particles used in the competitive LTIA of the present invention include polystyrene, a styrene-styrene sulfonate copolymer, a methacrylic acid polymer, an acrylic acid polymer, an acrylonitrile butadiene styrene copolymer, a vinyl chloride-acrylic acid ester copolymer and a polyvinyl acetate acrylate. The average particle size of the latex particles is appropriately selected from 50 µm to 400 µm in consideration of the concentration of the test substance in the test sample, the detection sensitivity of the measuring device, and the like.

### (Insoluble Carrier on which antigens or antibodies are immobilized)

In the present invention, as a method for immobilizing an antigen or antibody on an insoluble carrier such as latex particles, either physisorption (hydrophobic bonding) or chemical bonding can be appropriately selected according to the characteristics of the antigen or antibody to be immobilized. In the chemical bonding method, immobilization is possible by introducing a binding functional group such as a maleimide group into the antibody, or when the antigen or antibody has a sugar, by using it to bind to the binding functional group on the surface of the insoluble carrier.

### (Reagent for Competitive LTIA, Reagent Kit)

The composition outline of the reagent for competitive LTIA of the present invention is as described above, but a more specific composition will be described.

When immobilizing an antibody on latex, it is desirable to have a composition which includes at least the following (1) and (2), and
in which (1) and (2) are divided into a first reagent and a second reagent.

Note that while the examples of the following (1) and (2) included the case of being in a solution state, it is also possible to make a dried product at the time of storage, and in this case, it should be dissolved in a diluent or the like at the time of use to make it liquid. A case including a plurality of reagent compositions is sometimes referred to as a kit.
(1) A solution containing a polyvalent antigen in which a vitamin D derivative is immobilized on a carrier (vitamin D derivative-carrier complex) (sometimes referred to as a first reagent solution or the like)
(2) A latex particle solution on which anti-25(OH)VD antibodies are immobilized (sometimes referred to as a latex reagent solution, a second reagent solution, or the like)
   Furthermore, the reagent composition of the present invention sometimes includes the following (3), (4) and (5) .
(3) A standard substance for concentration conversion (sometimes referred to as a calibrator or the like)
(4) A solution for dissolving or diluting a standard substance for concentration conversion (sometimes referred to as a calibrator diluent or the like)
(5) A pretreatment liquid for releasing 25(OH)VD from vitamin D-binding protein

When the antigen is immobilized on latex, it includes at least the following (i) and (ii), and is otherwise the same as the case where an antibody is immobilized on the latex.
(i) A solution containing anti-25(OH)VD antibodies (sometimes referred to as the first reagent solution or the like)
(ii) A latex particle solution on which a vitamin D derivative is directly immobilized, or a latex particle solution on which an polyvalent antigen is immobilized in which a vitamin D derivative is immobilized on a carrier (sometimes referred to as a latex reagent solution, a second reagent solution, or the like)

### (Method for Measuring Agglutination Signal)

The measurement of the agglutination signal in the competitive LTIA may be any method as long as it is a method usually used for the measurement of the agglutination inhibition reaction, and includes the means that can be used by those skilled in the art such as the evaluation by the absorbance ratio, the measurement of number of particles, the measurement of particle size (the size increases when agglutinated), the measurement of scattered light or the measurement of the absorption spectrum (increases or shifts when agglutinated). Furthermore, optical detection can be replaced by electrochemical detection to the extent possible.

There are various methods for measuring the agglutination signal as described above, but a method using latex particles and a general-purpose biochemical analyzer is convenient. For example, it is possible to add a reagent containing a polyvalent antigen carrying a plurality of vitamin D derivatives and latex particles carrying anti-25(OH)VD antibodies to a sample containing the 25(OH)VD to be measured, and heat the mixture at a constant temperature for a certain period of time. The absorbance during this time can be then measured to detect the amount of change in absorbance, and the concentration of 25(OH)VD in the test sample can be calculated from a line using a standard solution whose concentration is previously known as a sample. In the latex turbidimetric immunoassay, the absorbance at a wavelength of 500 to 900 nm is usually used, and the amount of change in the absorbance during the reaction is generally used for quantification. The measurement range used in the present invention can be appropriately set to a desired measurement range in consideration of the type of measurement target, the avidity of the binding partner, the amount ratio of the binding partner to the polyvalent antigen, and the like.

The measurement of 25(OH)VD in the sample of the present invention may be performed by a manual method, or by using a device such as a measuring device. The measuring device may be a general-purpose automated analyzer or a dedicated measuring device (dedicated machine). In particular, the competitive LTIA can be carried out by a general-purpose automated analyzer, and can also be carried out by a method performed by a plurality of operation steps such as a two-step method (two-reagent method).

Hereinafter, the present invention will be further described in detail using examples, but the present invention is not limited thereto.

### Examples

### 1. Test Materials

The main materials and manufacturers used in the following examples are shown below.
BSA (Sigma-Aldrich)
Keyhole-Limpet Hemocyanin (Thermo Scientific)
Ovalbumin (Thermo Scientific)
Vitamin D2 (also known as Ergocalciferol) (Sigma-Aldrich) Vitamin D3 (also known as Cholecalciferol) (Sigma-Aldrich)
25-Hydroxy Vitamin D2 (Sigma-Aldrich)
25-Hydroxy Vitamin D3 (also known as Calcifediol) (FUJIFILM Wako Pure Chemical Corporation) ProClin300 (Sigma-Aldrich)
Dimethyl Sulfoxide (also known as DMSO) (FUJIFILM Wako Pure Chemical Corporation)

### [Test Example 1] Obtainment of 25(OH)VD3 Derivative

The 25(OH)VD3 derivative represented by the following chemical formula (IV) was obtained by outsourcing the synthesis to a contractor and used in the following test examples. [Formula 13]

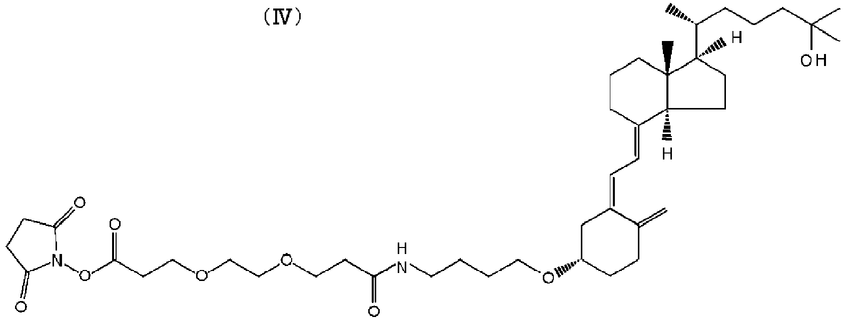

### [Test Example 2] Obtainment of Vitamin D3 Derivative

The vitamin D3 derivative represented by the following chemical formula (III) was obtained by outsourcing the synthesis to a contractor and used in the following test examples.

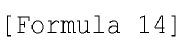

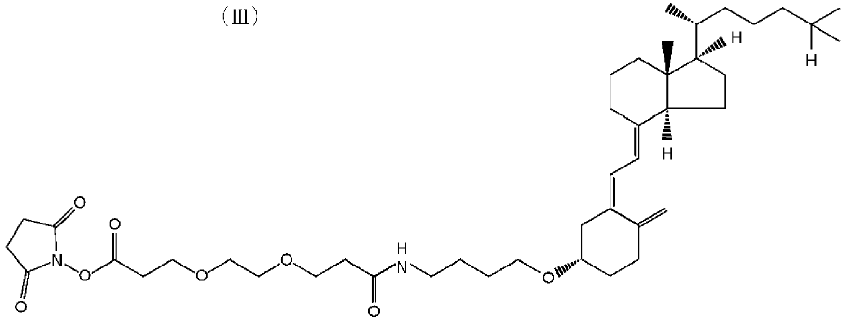

### [Example 1] Preparation of Vitamin D3 Derivative-BSA Complex

(1) Bovine serum albumin (BSA) was dissolved in 0.1 M Bicine buffer to obtain 1.0 mg/mL, and dialyzed with the same buffer.
(2) A vitamin D3 derivative was dissolved in dimethyl sulfoxide (DMSO) to obtain 10 mg/mL. This was added to the BSA solution of (1) in an amount of 1/30 (v/v), and the mixture was stirred by vortex.
(3) The mixture was incubated at room temperature for 2 hours under light shielding.
(4) 1M Tris-HCl pH 8.0 was added in an amount of 1/10 (v/v) of the total amount, and the mixture was stirred by vortex, then the reaction was stopped to obtain a vitamin D3 derivative-BSA complex.

### [Comparative Example 1] Preparation of 25(OH)VD3 Derivative-BSA Complex

A 25(OH)VD3 derivative-BSA complex was prepared in the same manner except that a 25(OH)VD3 derivative was used instead of a vitamin D3 derivative in Example 1(2).

### [Test Example 3] Obtainment of Anti-25(OH)VD3 Antibody

### 1. Obtainment of Antibody

A 25(OH)VD3 derivative was conjugated to keyhole-limpet hemocyanin, ovalbumin, or the like, and this was used as an immunogen to immunize mice by a conventional method. A final immunization was performed 3 to 4 days before cell fusion, then spleen cells and lymph node cells were collected and fused with myeloma cells (SP2/O) by electrofusion or PEG. The fused cells were cultured on a 96-well plate, and the culture supernatant was collected 7 to 8 days after the cell fusion and screened as shown below. The strains selected by screening were cloned and used for antibody purification.

### 2. Screening

### 2-1. Primary Screening

The strains reacting with the immobilized 25(OH)VD3 derivative were selected using the antigen-immobilized ELISA shown below.
(1) A 25(OH)VD3 derivative-BSA complex diluted to 1.0 µg/mL with PBS was dispensed as an immobilization antigen into a 96-well ELISA microplate in an amount of 50 µL/well, and allowed to stand at room temperature for 2 hours.
(2) After washing three times with 0.05% Tween 20-PBS (PBST) (400 µL/well), 1% BSA-PBST (100 µL/well) was dispensed as a blocking solution and the mixture was allowed to stand at room temperature for 1 hour.
(3) After removing the blocking solution, 50 µL/well of the culture supernatant was dispensed and allowed to stand at room temperature for 1 hour.
(4) After washing three times with PBST, 50 µL/well of HRP-labeled goat anti-mouse polyclonal antibody diluted 10000 times with a 1% BSA-PBST solution was dispensed and allowed to stand at room temperature for 1 hour.
(5) After washing three times with PBST, 50 µL/well of o-phenylenediamine chromogenic liquid was dispensed and allowed to stand at room temperature for 10 minutes.
(6) 50 µL/well of reaction terminator was dispensed, and the absorbance at a wavelength of 492 nm was measured to select the strains having a high absorbance.

### 2-2. Secondary Screening

The strains selected in the primary screening were further subjected to the competitive ELISA shown below, and the strains reacting with free 25(OH)VD2 and 25(OH)VD3 and not with free vitamin D2 and vitamin D3 were selected.
(1) A 25(OH)VD3 derivative-BSA complex diluted to 1.0 µg/mL with PBS was dispensed as an immobilization antigen into a 96-well ELISA microplate in an amount of 50 µL/well, and allowed to stand at room temperature for 2 hours.
(2) After washing three times with PBST, 1% BSA-PBST (100 µL/well) was dispensed as a blocking solution and the mixture was allowed to stand at room temperature for 1 hour.
(3) After removing the blocking solution, 25 µL/well of a solution in which vitamin D2, vitamin D3, 25(OH)VD2 or 25(OH)VD3 was dissolved in DMSO at 0, 0.1, 1, and 10 µg/mL was added as an inhibitory antigen. Furthermore, 25 µL/well of the culture supernatant was added and allowed to stand at room temperature for 1 hour.
(4) After washing three times with PBST, 50 µL/well of HRP-labeled goat anti-mouse polyclonal antibody diluted 10000 times with a 1% BSA-PBST solution was dispensed and allowed to stand at room temperature for 1 hour.
(5) After washing three times with PBST, 50 µL/well of o-phenylenediamine chromogenic liquid was dispensed and allowed to stand at room temperature for 10 minutes.
(6) 50 µL/well of reaction terminator was dispensed, and the absorbance at a wavelength of 492 nm was measured.
The strains having a high absorbance when free vitamin D2 and vitamin D3 were used as inhibitory antigens and a low absorbance when free 25(OH)VD2 and 25(OH)VD3 were used as inhibitory antigens were selected.

### 2-3. Results

A total of 6 hybridomas were obtained.

### [Example 2] Immunological Measurement

25(OH)VD3 was measured using the latex competitive inhibition method.

### I. Measurement Method and Procedure

### 1. Preparation of Reagent

### (1) Preparation of First Reagent

A first reagent having the following composition containing 0.1 µg/mL of the vitamin D3 derivative-BSA complex prepared in Example 1 was prepared.

### [Composition of First Reagent]

100 mM Bis-Tris pH 7.0
300 mM NaCl
0.05% ProClin300
0.5% BSA
0.1 µg/mL Vitamin D3 Derivative-BSA Complex

### (2) Preparation of Second Reagent

To 1.0 mL of a buffer solution containing 0.36 mg of the anti-25(OH)VD3 antibodies obtained in Test Example 3, 1.0 mL of a 1% latex (manufactured in-house) suspension having an average particle size of about 350 nm was added, and the mixture was stirred at 4°C for 2 hours. Subsequently, 1.0 mL of a buffer solution containing 0.1% BSA was added, and the mixture was stirred at 4°C for 1 hour. A second reagent having the following composition was prepared by filtering the obtained antibodysensitized latex solution through a filter having a pore size of 0.8 µm and then diluting to 3.0 OD at a wavelength of 600 nm.

### [Composition of Second Reagent]

5 mM MOPS-NaOH pH 7.0
3.0 OD (600nm) Anti-25(OH)VD3 Antibody Sensitized Latex
0.05% ProClin300

### (3) Preparation of 25(OH)VD3 dilution series

25(OH)VD3 was dissolved in DMSO to prepare a 750 nM solution. Furthermore, the present product was diluted with DMSO to prepare 375, 188, 94, 47 and 23 nM solutions. These were referred to as 25(OH)VD3 dilution series and used as samples in the following measurement.

### 2. Measurement Method

A Hitachi 7170 automated analyzer was used for the measurement. 3.0 µL of the samples and 100 µL of the first reagent were added to the reaction cells and reacted at 37°C for 5 minutes. Furthermore, 100 µL of the second reagent was added to the reaction cells and reacted at 37°C for 5 minutes, and the amount of change in absorbance was measured by a 2 point end method (photometric point 19-34) at a main wavelength of 570 nm.

### II. Measurement Results

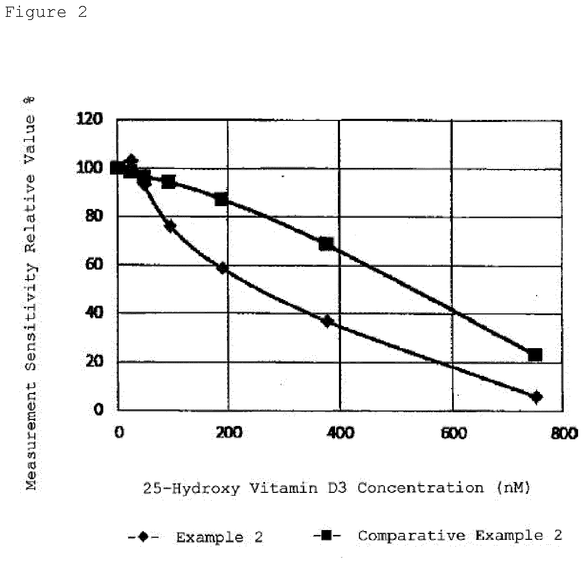

The 25(OH)VD3 dilution series was measured as the samples, and DMSO was measured as the negative control (zero 25(OH)VD3 concentration). The measurement sensitivities (mAbs.) in each dilution series of 25(OH)VD3 are shown in Table 2. In addition, the relative sensitivity (%) of each dilution series with respect to zero concentration is shown in Figure 2.

### [Comparative Example 2]

The reagent was prepared in the same manner as in "Preparation of Reagent" of Example 2-1-1, except that a 25(OH)VD3 derivative-BSA complex was added to the first reagent as the vitamin D3 derivative-BSA complex, and the measurement was performed in the same manner as in "Measurement method" of Example 2-2. The measurement results are shown in Table 2 and Figure 2 together with the results of Example 2.

### [Table 2]

**Table 2. Measurement Results of 25(OH)VD3 Dilution Series (Sensitivity mAbs.)**

| | | Measurement Sensitivity (mAbs.) | |
|---|---|---|---|
| | | Example 2 | Comparative Example 2 |
| | | Vitamin D3 | 25-Hydroxy Vitamin D3 |
| Concentration of 25-Hydroxy Vitamin D3 Dilution Series (nM) | 0 | 241 | 231 |
| | 23 | 248 | 228 |
| | 47 | 224 | 223 |
| | 94 | 184 | 218 |
| | 188 | 142 | 202 |
| | 375 | 89 | 159 |
| | 750 | 14 | 54 |

### [Discussion]

From Figure 2, it can be read that the concentration of 25(OH)VD3 required to reduce the measurement sensitivity by 10% compared to when 25(OH)VD3 is 0 nM, was about 55 nM in Example 2, but it was about 150 nM in Comparative Example 2, with a measurement sensitivity approximately tripled.

Therefore, this demonstrates that when a vitamin D3 derivative-BSA complex is used as the polyvalent antigen of the reagent for measuring 25(OH)VD by the competitive inhibition method, the degree of competitive reaction is higher than when a 25(OH)VD3 derivative-BSA complex is used and a highly sensitive detection is possible.

### Industrial Applicability

According to the present invention, it has become possible to provide a measurement method and a measurement reagent based on a competitive immunoassay having good sensitivity even when the 25(OH)VD concentration in a sample is low. In addition, when the present invention is applied to competitive LTIA, it is possible to measure 25(OH)VD of a large number of samples in a short time by applying them to an automated analyzer which is a general-purpose equipment.

## Claims

1. A reagent for measuring 25-hydroxy vitamin D based on a competitive immunoassay, comprising at least the following composition:
(1) a vitamin D derivative represented by the following chemical formula (I) and/or (II); and
(2) an anti-25 hydroxy vitamin D antibody, 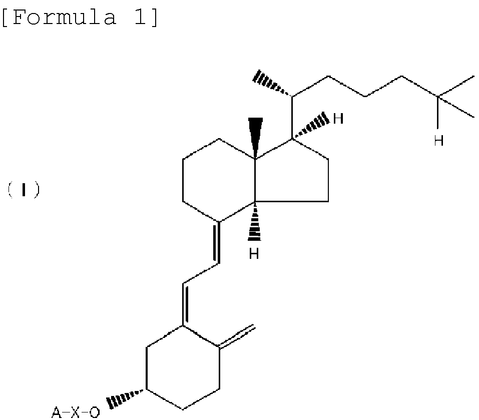 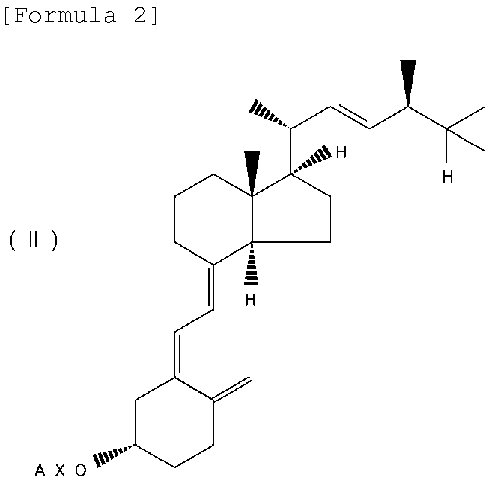
wherein A represents a tracer group capable of chemically binding to a carrier with high affinity, and
X indicates a hydrocarbon group having a chain length of 3 to 20, which is unsubstituted or substituted with a hetero atom.

2. The reagent for measuring 25-hydroxy vitamin D according to claim 1, wherein the A is selected from an amino group, a carboxyl group, a sulfhydryl group, biotin, digoxygenin, tyrosine, FITC-substituted tyrosine, substituted amino acids, amino acid and peptide sequences, FITC, proteins and peptides, A-proteins, G-proteins, and vitamin D derivatives.

3. The reagent for measuring 25-hydroxy vitamin D according to claim 1 or 2, wherein the vitamin D derivative represented by the chemical formula (I) or (II) is in a configuration in which two or more molecules bind to a carrier via A and form a polyvalent antigen.

4. The reagent for measuring 25-hydroxy vitamin D according to any one of claims 1 to 3, wherein the measurement principle of the competitive immunoassay is a competitive immunoassay selected from RIA, EIA, LTIA, and CLEIA.

5. The reagent for measuring 25-hydroxy vitamin D according to claim 4, wherein the competitive immunoassay is competitive latex turbidimetric immunoassay (competitive LTIA).

6. The reagent for measuring 25-hydroxy vitamin D according to any one of claims 1 to 5, wherein 25-hydroxy vitamin D is the sum of 25-hydroxy vitamin D2 and 25-hydroxy vitamin D3, the vitamin D derivative is a vitamin D2 derivative and/or vitamin D3 derivative, and the anti-25-hydroxy vitamin D antibody is an anti-25-hydroxy vitamin D2 and/or anti-25-hydroxy vitamin D3 antibody.

7. The reagent for measuring 25-hydroxy vitamin D according to any one of claims 1 to 6, wherein the vitamin D3 derivative is a compound represented by the following chemical formula (III):

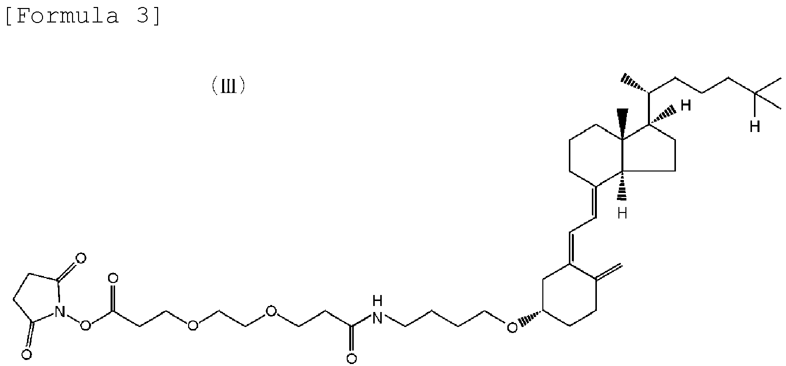

8. The reagent for measuring 25-hydroxy vitamin D according to any one of claims 1 to 7, wherein either (1) or (2) is immobilized on an insoluble carrier.

9. The reagent for measuring 25-hydroxy vitamin D according to any one of claims 1 to 8, which is a reagent for an automated analyzer.

10. A method for measuring 25-hydroxy vitamin D based on a competitive immunoassay, comprising at least the following steps:
(1) a step of bringing a sample into contact with anti-25-hydroxy vitamin D antibodies in the presence of a vitamin D derivative represented by the chemical formula (I) and/or (II) : 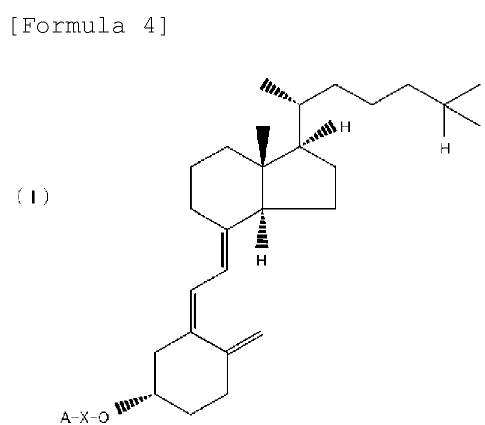 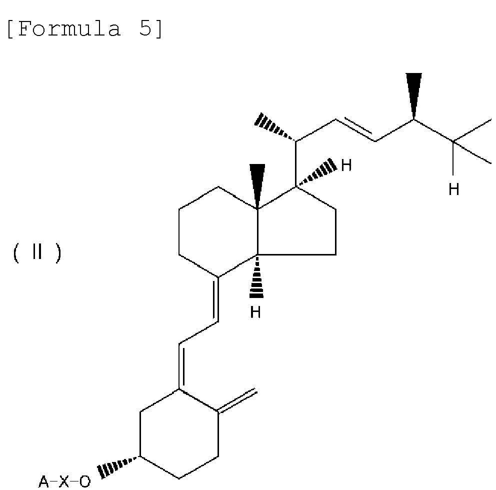
wherein A represents a tracer group capable of chemically binding to a carrier with high affinity; and
X indicates a hydrocarbon group having a chain length of 3 to 20, which is unsubstituted or substituted with a hetero atom; and
(2) a step of measuring the degree of inhibition of the antigen-antibody reaction between the vitamin D derivative and the anti-25-hydroxy vitamin D antibodies according to the 25-hydroxy vitamin D in the sample.

11. The method for measuring 25-hydroxy vitamin D according to claim 10, wherein A is selected from an amino group, a carboxyl group, a sulfhydryl group, biotin, digoxygenin, tyrosine, FITC-substituted tyrosine, substituted amino acids, amino acid and peptide sequences, FITC, proteins and peptides, A-proteins, G-proteins, and vitamin D derivatives.

12. The method for measuring 25-hydroxy vitamin D according to claim 10 or 11, wherein the vitamin D derivative represented by the chemical formula (I) or (II) is in a configuration in which two or more molecules bind to a carrier via A and form a polyvalent antigen.

13. The method for measuring 25-hydroxy vitamin D according to any one of claims 10 to 12, wherein the measurement principle of the competitive immunoassay is a competitive immunoassay selected from RIA, EIA, LTIA, and CLEIA.

14. The method for measuring 25-hydroxy vitamin D according to claim 13, wherein the competitive immunoassay is competitive latex turbidimetric immunoassay (competitive LTIA).

15. The method for measuring 25-hydroxy vitamin D according to any one of claims 10 to 14, wherein 25-hydroxy vitamin D is the sum of 25-hydroxy vitamin D2 and 25-hydroxy vitamin D3, the vitamin D derivative is a vitamin D2 derivative and/or vitamin D3 derivative, and the anti-25-hydroxy vitamin D antibody is anti-25-hydroxy vitamin D2 and/or anti-25-hydroxy vitamin D3 antibody.

16. The method for measuring 25-hydroxy vitamin D according to any one of claims 10 to 15, wherein the vitamin D3 derivative is a compound represented by the following chemical formula (III):

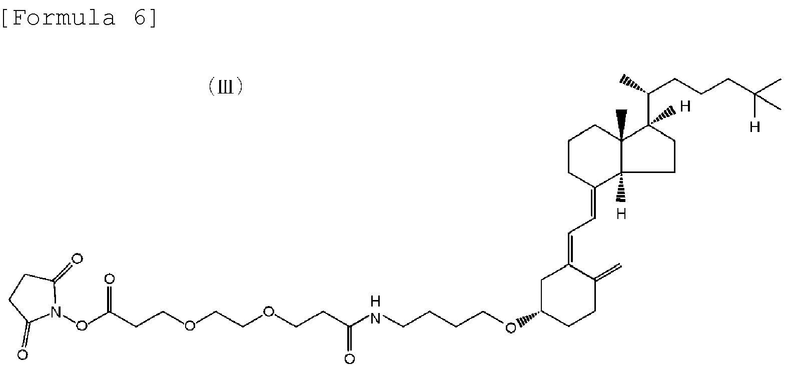

17. The method for measuring 25-hydroxy vitamin D according to any one of claims 10 to 16, wherein either (1) or (2) is immobilized on an insoluble carrier.

18. The method for measuring 25-hydroxy vitamin D according to any one of claims 10 to 17, which uses an automated analyzer.

19. A polyvalent antigen used in the method for measuring 25-hydroxy vitamin D based on a competitive immunoassay, wherein a vitamin D3 derivative represented by the following chemical formula (III) is immobilized on a carrier:
